(19) **Europäisches Patentamt**

**European Patent Office**

**Office européen des brevets**

(11) **EP 0 990 878 B1**

(12) **EUROPEAN PATENT SPECIFICATION**

(45) Date of publication and mention
of the grant of the patent:
**07.05.2003 Bulletin 2003/19**

(51) Int Cl.[7]: **G01F 23/00**, G01N 33/14,
G01F 23/24

(21) Application number: **99202998.3**

(22) Date of filing: **15.09.1999**

(54) **Method and apparatus for determining the stability of a layer of foam**

Verfahren und Vorrichtung zum Ermitteln der Stabilität einer Schaumschicht

Méthode et dispositif pour déterminer la stabilité d'une couche de mousse

(84) Designated Contracting States:
**AT BE CH CY DE DK ES FI FR GB GR IE IT LI LU
MC NL PT SE**

(30) Priority: **29.09.1998 NL 1010213**

(43) Date of publication of application:
**05.04.2000 Bulletin 2000/14**

(73) Proprietor: **Haffmans B.V.
NL-5902 RD Venlo (NL)**

(72) Inventor: **Van Akkeren, Franciscus Johannes
Jacobus
6039 ES Stramproy (NL)**

(74) Representative: **Valkonet, Rutger et al
Algemeen Octrooibureau
P.O. Box 645
5600 AP Eindhoven (NL)**

(56) References cited:
**EP-A- 0 240 432          GB-A- 2 161 601
NL-A- 7 605 981          US-A- 4 059 016
US-A- 4 677 304**

**Description**

The invention relates to a method for determining the stability of a layer of foam on a liquid column, for example beer, by measuring the time interval during which a surface of the layer of foam moves a certain distance under particular circumstances, which time interval is subsequently compared with a reference time interval associated with said distance.

The invention furthermore relates to an apparatus for carrying out the method according to the invention.

The stability of a layer of foam on a liquid column, for example the head on beer, contributes to a large extent to the subjective quality perception of the beer by the consumer. In order to meet this subjective perception by the consumer, beer breweries use a more or less standardised method for testing the stability of the layer of foam on their beer. Beer breweries thereby measure the time interval during which the upper surface of the layer of foam subsides over a standardised distance and under predetermined ambient conditions or during which the lower foam surface (liquid-foam transition) rises. The measured time interval of the type of beer in question is compared with a reference time interval set up by the brewery for internal quality control. If the measured time interval is smaller than this reference interval, the brewery is of the opinion that the layer of foam does not meet the subjective stability requirements which the brewery has established for the benefit of the public. The brewery will then have to check the brewery process and/or the raw materials for impurities or irregularities and possibly make adjustments with regard to the brewing process or the raw materials, so that the beer currently being brewed and/or the beer to be brewed in the future will meet said stability requirements.

When an excessive deviation is found to occur between the measured time interval and the reference time interval determined on subjective assessment grounds, brewers only think of possible irregularities in the brewing process when looking for the cause of this deviation. Any external influences on the movement of the surface of a layer of foam on a beer column during the measurement are not taken into account, however. This may lead to a situation wherein a deviation in the measured time interval cannot be owed to an irregularity in the brewing process, but completely to the environment in which the stability test is carried out. Such an incorrect measurement may thus lead to a batch of beer being declared unfit without good reason and also to unnecessary standstill and troubleshooting effort with regard to the brewing process.

The object of the invention is to overcome the above drawback and to provide a method and an apparatus for determining the stability of a layer of foam on a liquid column which, unlike the current methods and apparatus, also takes into account the influence of the environment on the stability of the foam layer during the measurement of the time interval.

In order to accomplish that objective, the method according to the invention is characterized in that at least one external environmental quantity is registered during the movement of the foam surface and that after the time interval has been measured the registered external environmental quantity is used for correcting the measured time interval. By correcting the measured time interval for the influence of at least one external environmental quantity, a measuring value for the foam stability is obtained which not only is more accurate but which moreover prevents incorrect interpretation of the measured time interval. Since the measured time interval cannot be incorrectly interpreted, the possibility of a batch of newly brewed beer being declared unfit without good reason is reduced to a minimum and unnecessary standstill of the brewing process is prevented.

An highly accurate measuring result and a correct correction of the measurement for an external environmental quantity can be obtained in that according to the invention the correlation between the time interval and each external environmental quantity is determined and that the measured time interval is corrected for said external environmental quantity on the basis of said correlation.

According to the invention, a possible correction may thereby be characterized in that said correlation exhibits a linear relationship and in that the measured time interval for said external environmental quantity is corrected by a factor which equals the product of the deviation of the measured value of said external environmental quantity from a reference value of said environmental quantity with the determined correlation.

The invention furthermore relates to an apparatus for determining the stability of a layer of foam on a liquid column, for example beer, by measuring the time interval during which a surface of the layer of foam moves a certain reference distance, said apparatus comprising a housing which contains means for registering said time interval and said reference distance and means for displaying the results obtained upon said registration, as well as at least one detector which detects the level of the foam surface.

An apparatus of this kind is generally known, it is marketed by the applicant of this patent application. The apparatus is thereby arranged for measuring the time interval during which the upper surface of the layer of foam subsides over the reference distance. The measurement of a time interval requires detection of the level of the subsiding foam surface. To this end, two vertically movable detectors in the form of electrodes are positioned above the layer of foam, whereby the exact level of the upper foam surface can be detected on the basis of the electrical contact between the two electrodes via the foam surface. When the electrical contact between the two electrodes is broken upon further subsidence of the layer of foam, moving and positioning means ensure that the electrodes come into contact with the foam surface again. The time interval during which the electrodes (and the foam surface) have been moved a certain distance is a measure for the stability of the layer of foam. The drawback of the prior apparatus, however, is the fact that it is not possible to deduce unequivocally from the detected deviation of the measured time interval whether said deviation is caused by irregularities in the brewing process or by other factors, for example external influences.

According to the invention, the apparatus is characterized in that said apparatus furthermore includes one or more sensors for registering one or more external environmental quantities for the purpose of correcting the measured time interval for said environmental quantity.

In one embodiment of the apparatus according to the invention, which enables a more accurate stability test of a layer of foam, a temperature sensor is present on the free end of at least one electrode for the purpose of registering the temperature of the layer of foam.

In another embodiment, the detector is an optical sensor which is disposed beside the layer of foam. The detector may also be in the form of an optical or an ultrasonic detector, which is disposed above the layer of foam. These embodiments enable contactless measurement, whereby the layer of foam is not disturbed.

According to the invention, the sensor may be in the form of a temperature sensor, an air pressure sensor, an air humidity sensor or a velocity sensor for the air circulation. The provision of such sensors in the apparatus according to the invention makes it possible to subject the measurement to a correction, as a result of which the apparatus provides more accurate and reliable measuring results. The risk of incorrect conclusions being drawn on the basis of such measurements is thus strongly reduced.

The invention will now be explained in more detail with reference to the drawing, which successively shows in:

    Figure 1 an embodiment of the apparatus according to the invention; and in
    Figures 2a and 2b the apparatus of Figure 1 while carrying out the method according to the invention.

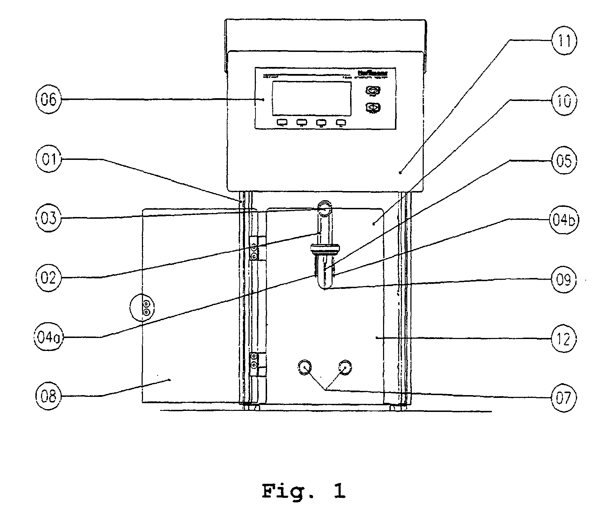

Figure 1 shows an embodiment of the apparatus according to the invention, which is arranged for measuring the time interval during which the upper foam surface subsides over a certain reference distance. The apparatus includes a housing 1 provided with a slot 2, from which a support 3 extends. Support 3 can be moved in vertical direction in slot 2 by suitable moving means accommodated in housing 1 (not shown). Support 3 is provided with at least two, three in this embodiment, detectors 4a, 4b and 5 in the form of electrodes. The construction of four electrodes 4a-4d arranged round central electrode 5 provides an accurate measurement, however. Said electrodes are connected, via support 3, to measuring and control means 3 (likewise not shown) accommodated in housing 1. The apparatus furthermore includes a display and input panel 6, via which panel the driving, measuring and control means present in housing 1 can be operated.

A stability test on a layer of foam on a liquid column is carried out as follows. A graduated beaker of standardized dimensions (not shown) is filled with a predetermined amount of foam to be examined. The graduated beaker is placed into a space 12 formed by housing 1, in abutment with locating and positioning supports 7. Support 3 with sensors 4a, 4b and 5 is disposed above the layer of foam created on the beer column. The driving means accommodated in housing 1 moves support 3 in vertical direction until sensor 5 extends into the layer of foam and sensors 4a and 4b just touch the surface of the layer of foam. The exact level of the layer of foam can now be established by means of the electrical contact which is effected between sensors 4a, 4b and sensor 5 via the foam surface. This level of the layer of foam is regarded as the starting point for the test.

The layer of foam on a beer column will start to subside after some time, with $CO_2$ diffusing from the beer into the environment via the layer of foam. To determine the stability of the layer of foam, the time interval is measured during which the surface of a layer of foam subsides over certain, predetermined distance. After the test, this measured time interval is compared with a reference time interval which the beer brewer has imposed upon himself as a quality standard under the assumption of a number of reference conditions.

When the measured time interval deviates too much from the reference time interval, this is currently still considered to be a consequence of certain irregularities in the brewing process. The present measurement can thus enable a quick assessment of the stability of the layer of foam and of the quality of the beer.

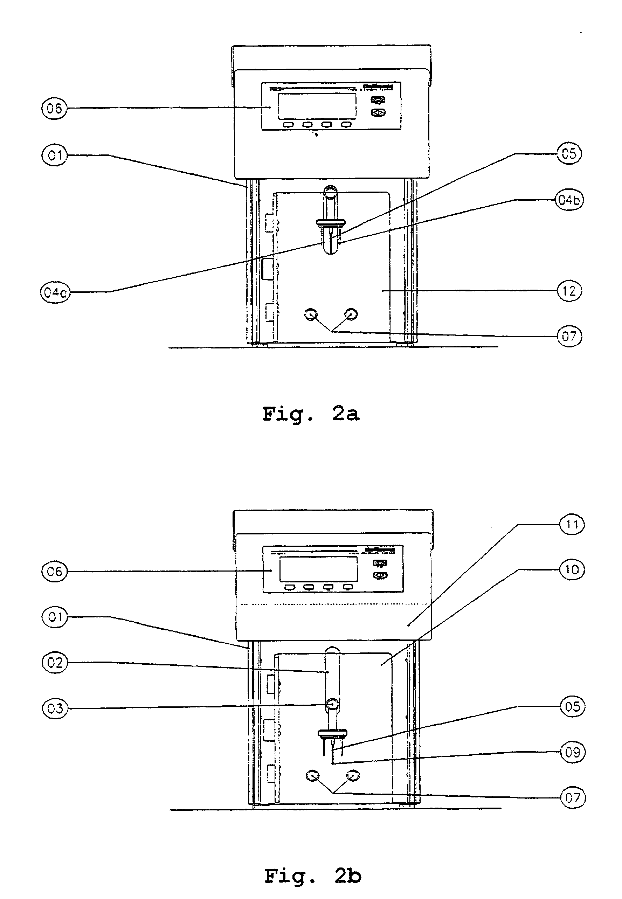

In order to measure said time interval, the measuring means of the apparatus monitors the electrical contact between sensors 4a, 4b via the foam surface to sensor 5. When the electrical contact is broken due to the subsidence of the layer of foam, the driving apparatus will move support 3 a little further downwards (see Figures 2a and 2b) until the electrical contact is restored again. During said subsiding of the layer of foam, support 3 will thus move a predetermined distance. The period of time during which support 3 is moved said distance is measured and displayed on panel 6. The user can read this measured time interval and compare it with the reference standard which the brewer has imposed upon himself.

The apparatus according to the invention uses the insight on which the method according to the invention is based. This insight relates to the registration of the correlation or the influence of one or more external environmental quantities on the time interval during which the surface of the layer of foam moves the predetermined reference distance.

A few external environmental quantities which have been found to be capable of influencing the measurement of the time interval to a significant degree during a quality test are for example the temperature of the liquid column, the temperature of the layer of foam, the ambient temperature, the air pressure, the relative humidity above the liquid column, as well as the degree of air circulation round the electrodes and the layer of foam. The individual correlations between the time interval and said external environmental quantities are laid down for each type of beer and they are used with the method according to the invention for correcting the measured time interval for the influence of said external environmental quantity.

In some cases said correlation exhibits a more or less linear relationship, and it exhibits a substantially linear relationship round a certain reference value of the external environmental quantity. It is very well possible, however, that the correlation does not exhibit a linear relationship, but for example a logarithmic, exponential, second-degree or other relationship. Under such circumstances the correction of the measured time interval will have to take place on the basis of said desired correlation. Purely by way of example, the following description will relate to the measured time interval on the basis of the correlation(s) between the time interval of one or more external environmental quantities which exhibit(s) a linear relationship.

Not only the correct correlation associated with the particular type of beer is used during the quality test, but also the instantaneous value of the external environmental quantity for which the measured time interval must be corrected is measured. To that end, the apparatus according to the invention includes one or more sensors 9, 10 and 11, by means of which the respective environmental quantity is registered. A very specific embodiment is shown in Figure 1, it relates to the placing of a sensor 9 on the free end of electrode 5. Said sensor 9 is in the form of a temperature sensor and it measures the temperature of the layer of foam, since the electrode extends into the layer of foam during the test. The temperature of the layer of foam is partially influenced by the temperature of the beer present thereunder, as well as by the ambient temperature, and it can be regarded to be one of the main factors that influence the stability of the layer of foam.

After the uncorrected time interval has been measured, the measured value is corrected with a factor which equals the product of the deviation of the value measured via sensor 9 relative to a pre-set reference foam temperature with the predetermined correlation. Said (linear) correction is represented by the following formula

$$t_C = t_M + (k_{M,1} - k_{R,1}) \times c_{b,1} \qquad [1]$$

wherein

| | |
|---|---|
| $t_C$ | is the corrected time interval [s], |
| $t_M$ | the measured time interval [s], |
| $k_{M,1}$ | the measured value of external environmental quantity 1 [?], |
| $k_{R,1}$ | the reference value of external environmental quantity 1 [?], |
| $c_{b,1}$ | the correlation factor between the time interval and external environmental quantity 1 for beer type b [s/?]. |

The ?-sign refers to the S.I. unit of the external environmental quantity used for the correction. The influence of an external environmental quantity on the subsidence of the layer of foam is corrected by the corrected measurement of the time interval with a very good approximation. Any differences between the measured, now corrected time interval and the reference time interval can now be traced back with very great probability to irregularities that have occurred during the brewing process.

Repeated measurements of the time interval in accordance with the standard method and in accordance with the inventive method have shown that the (corrected) measurements made in accordance with the method according to the invention exhibit a slighter scattering and a smaller standard deviation. From this it can be concluded that the measuring results obtained by using the method according to the invention provide a more accurate and more reliable picture of the quality of the layer of foam to be tested.

Sensor 10 may for example be a velocity meter, which detects the air circulation round electrodes 4a, 4b and 5 and above the layer of foam. Air circulation or other air flows may for example be produced by climate control or ventilation devices, which are present in the test chamber, but also by movements made by the operating staff. The air flow strongly influences the gaseous diffusion of $CO_2$ from the beer through the foam into the environment. A further reduction of the air circulation can be obtained by closing space 12 in which the liquid column to be tested is present by means of a covering 8.

Sensor 11 may for example be used for measuring the ambient air pressure. In the case of a high ambient air pressure, the gaseous diffusion of $CO_2$ from the beer through the layer of foam into the environment will take place less quickly, as a result of which the foam will subside more quickly.

In addition to that, a sensor may be used for registering the relative humidity of the environment. A lower air humidity level accelerates the evaporation process of the beer, as a result of which the foam will subside more quickly.

However, the correction of the measured time interval for the temperature is one of the most important applications of the method according to the invention. The temperature influences in particular the viscosity of the layer of foam as well as the gaseous diffusion of $CO_2$ from the beer through the layer of foam into the environment.

It will be understood that the use of the method and the apparatus according to the invention provide a more accurate picture of the quality of a layer of foam which is present on a beer column. Although in the present disclosure, the measured value of the time interval is only corrected for the influence of one environmental quantity, for example the foam temperature, it is very well possible to correct the measured value of the time interval for more than one external environmental quantity. In such cases similar correction factors must be placed after the correction factor for external environmental quantity 1 as shown in formula 1. In general, the correction factor will then be built up of several linear corrections for each external environmental quantity,

$$t_C = t_M + \Sigma\ (k_{M,n} - k_{R,n}) \times c_{b,n} \qquad [2]$$

wherein n is the $n^{th}$ external environmental quantity for which the measurement is corrected.

It will be quite obvious that when the correlations between the time interval and one or more external environmental quantities do not exhibit a linear relationship, the form of the correction terms in the correction formulas [1] and [2] must accordingly be formulated differently. In addition to that it is possible to include in the correction operation correction terms which describe the influence of an external environmental quantity on another external environmental quantity.

The comparison of the measured and/or the corrected time interval with the reference time interval can possibly be carried out automatically by the control means of the apparatus. To this end, the reference time interval must be stored in the control means. Possibly, the apparatus can be driven and controlled via a PC.

The apparatus according to the invention is not only suitable for measuring and correcting the time interval during the subsidence of the upper surface of the layer of foam. The apparatus may also be used for carrying out the measurement with regard to the upward movement of the lower surface of the layer of foam, that is, with regard to the moving liquid-foam transition of the beer column. In that case sensor 9 on electrode 5 will register the temperature of the beer column.

Furthermore it will be quite obvious that the method and apparatus according to the invention not only are suitable for carrying out stability tests on beer foam, but that it is also possible to subject the layer of foam on other sparkling liquids, such as champagne, to such a test.

**Claims**

1. A method for determining the stability of a layer of foam on a liquid column, for example beer, by measuring the time interval during which a surface of the layer of foam moves a certain distance under particular circumstances, which time interval is subsequently compared with a reference time interval associated with said distance, **characterized in that** at least one external environmental quantity is registered during the movement of the foam surface and that after the time interval has been measured the registered external environmental quantity is used for correcting the measured time interval.

2. A method according to claim 1, **characterized in that** the correlation between the time interval and each external environmental quantity is determined and that the measured time interval is corrected for said external environmental quantity on the basis of said correlation.

3. A method according to claim 2, **characterized in that** said correlation exhibits a linear relationship and **in that** the measured time interval for said external environmental quantity is corrected by a factor which equals the product of the deviation of the measured value of said external environmental quantity from a reference value of said environmental quantity with the determined correlation.

4. A method according to any one of the preceding claims, **characterized in that** the temperature of the liquid column, the temperature of the layer of foam, the ambient temperature, the air pressure, the relative humidity above the liquid column or the degree of air circulation are used as one of the external environmental quantities for the correction of the time interval.

5. Apparatus for determining the stability of a layer of foam on a liquid column, for example beer, by measuring the time interval during which a surface of the layer of foam moves a certain reference distance, said apparatus comprising a housing which contains means for registering said time interval and said reference distance and means for displaying the results obtained upon said registration, as well as at least one detector which detects the level of the foam surface, **characterized in that** said apparatus furthermore includes one or more sensors for registering one or more external environmental quantities for the purpose of correcting the measured time interval for said environmental quantity.

6. Apparatus according to claim 5, wherein said housing is also provided with a vertically movable support with at least two detectors in the form of electrodes, which detect the level of the foam surface according to the principle of electrical conduction, as well as moving and positioning means for said support and said electrodes, **characterized in that** a temperature sensor is disposed near the free end of at least one electrode for registering the temperature of the layer of foam.

7. Apparatus according to claim 5, **characterized in that** said detector is in the form of an optical detector, which is disposed beside the layer of foam.

8. Apparatus according to claim 5, **characterized in that** said detector is in the form of an optical detector or an ultrasonic detector, which is disposed above the layer of foam.

9. Apparatus according to any one of the claims 5 - 8, **characterized in that** said sensor is a temperature sensor, an air pressure sensor, an air humidity sensor or a velocity sensor for the air circulation.

**Patentansprüche**

1. Verfahren zum Bestimmen der Stabilität einer Schaumschicht auf einer Flüssigkeitssäule, beispielsweise Bier, durch Messen des Zeitintervalls, währenddessen eine Oberfläche der Schaumschicht sich eine bestimmte Strecke unter besonderen Umständen bewegt, wobei das Zeitintervall nachfolgend mit einem Referenzzeitintervall, das zu dieser Strecke gehört, verglichen wird, **dadurch gekennzeichnet, dass** mindestens ein externer Umgebungsparameter während der Bewegung der Schaumoberfläche erfasst wird und dass, nachdem das Zeitintervall gemessen wurde, der erfasste externe Umgebungsparameter verwendet wird, um das gemessene Zeitintervall zu korrigieren.

2. Verfahren nach Anspruch 1, **dadurch gekennzeichnet, dass** die Korrelation zwischen dem Zeitintervall und jedem externen Umgebungsparameter bestimmt wird und dass das gemessene Zeitintervall für den externen Umgebungsparameter auf der Basis der Korrelation korrigiert wird.

3. Verfahren nach Anspruch 2, **dadurch gekennzeichnet, dass** die Korrelation ein lineares Verhältnis aufweist und dass das gemessene Zeitintervall für den externen Umgebungsparameter mit einem Faktor korrigiert wird, der dem Produkt der Abweichung des gemessenen Werts des externen Umgebungsparameters von einem Referenzwert des Umgebungsparameters mit der bestimmten Korrelation entspricht.

4. Verfahren nach einem der vorhergehenden Ansprüche, **dadurch gekennzeichnet, dass** die Temperatur der Flüssigkeitssäule, die Temperatur der Schaumschicht, die Umgebungstemperatur, der Luftdruck, die relative Feuchtigkeit über der Flüssigkeitssäule oder das Maß der Luftzirkulation als einer der externen Umgebungsparameter für die Korrektur des Zeitintervalls verwendet werden.

5. Vorrichtung zum Bestimmen der Stabilität einer Schaumschicht auf einer Flüssigkeitssäule, beispielsweise Bier, durch Messen des Zeitintervalls, währenddessen sich eine Oberfläche der Schaumsicht eine bestimmte Referenzstrecke bewegt, wobei die Vorrichtung ein Gehäuse umfasst, das eine Einrichtung zum Erfassen des Zeitintervalls und der Referenzstrecke enthält, und eine Einrichtung zum Darstellen der Ergebnisse, die durch die Erfassung erhalten werden, sowie mindestens einen Detektor, der das Niveau der Schaumoberfläche erfasst, **dadurch gekennzeichnet, dass** die Vorrichtung ferner einen oder mehreren Sensoren zum Erfassen von einem oder mehreren externen Umgebungsparametern zum Zweck des Korrigierens des gemessenen Zeitintervalls für den Umgebungsparameter umfasst.

6. Vorrichtung nach Anspruch 5, wobei das Gehäuse auch mit einem vertikal bewegbaren Träger mit mindestens zwei Detektoren in der Gestalt von Elektroden, die das Niveau der Schaumoberfläche nach dem Prinzip der elektrischen Leitfähigkeit erfassen, sowie einer Bewegungs- und Positioniereinrichtung für den Träger und die Elektroden versehen ist, **dadurch gekennzeichnet, dass** ein Temperatursensor in der Nähe des freien Endes mindestens einer Elektrode angebracht ist, um die Temperatur der Schaumschicht zu erfassen.

7. Vorrichtung nach Anspruch 5, **dadurch gekennzeichnet, dass** der Detektor in der Gestalt eines optischen Erfassers ist, der neben der Schaumschicht angebracht ist.

8. Vorrichtung nach Anspruch 5, **dadurch gekennzeichnet, dass** der Detektor in der Gestalt eines optischen Erfassers oder eines Ultraschallerfassers ist, der über der Schaumschicht angebracht ist.

9. Vorrichtung nach einem der Ansprüche 5 bis 8, **dadurch gekennzeichnet, dass** der Sensor ein Temperatursensor ist, ein Luftdrucksensor, ein Luftfeuchtigkeitssensor oder ein Geschwindigkeitssensor für die Luftzirkulation.

## Revendications

**1.** Procédé de détermination de la stabilité d'une couche de mousse sur une colonne de liquide, par exemple, de bière, en mesurant l'intervalle de temps pendant lequel une surface de la couche de mousse se déplace sur une certaine distance dans des conditions particulières, lequel intervalle de temps est ensuite comparé à un intervalle de temps de référence associé à ladite distance, **caractérisé en ce qu'**au moins une grandeur d'environnement externe est enregistrée au cours du déplacement de la surface de mousse et **en ce qu'**après la mesure de l'intervalle de temps, la grandeur d'environnement externe est utilisée afin de corriger l'intervalle de temps mesuré.

**2.** Procédé selon la revendication 1, **caractérisé en ce que** la corrélation entre l'intervalle de temps et chaque grandeur d'environnement externe est déterminée et **en ce que** l'intervalle de temps mesuré est corrigé en fonction de ladite grandeur d'environnement externe sur la base de ladite corrélation.

**3.** Procédé selon la revendication 2, **caractérisé en ce que** ladite corrélation présente une relation linéaire et **en ce que** l'intervalle de temps mesuré en fonction de ladite grandeur d'environnement externe est corrigé d'un facteur qui est égal au produit de l'écart de la valeur mesurée de ladite grandeur d'environnement externe par rapport à une valeur de référence de ladite grandeur d'environnement avec la corrélation déterminée.

**4.** Procédé selon l'une quelconque des revendications précédentes, **caractérisé en ce que** la température de la colonne de liquide, la température de la couche de mousse, la température ambiante, la pression d'air, l'humidité relative au-dessus de la colonne de liquide ou le niveau de circulation d'air sont utilisés comme l'une des grandeurs d'environnement externe pour la correction de l'intervalle de temps.

**5.** Dispositif destiné à déterminer la stabilité d'une couche de mousse sur une colonne de liquide, par exemple, de bière, en mesurant l'intervalle de temps au cours duquel une surface de la couche de mousse se déplace d'une certaine distance de référence, ledit dispositif comprenant à boîtier qui contient un moyen destiné à enregistrer ledit intervalle de temps et ladite distance de référence et un moyen destiné à afficher les résultats obtenus lors dudit enregistrement, de même qu'au moins un détecteur qui détecte le niveau de la surface de mousse, **caractérisé en ce que** ledit dispositif comprend, en outre, un ou plusieurs capteurs destinés à enregistrer une ou plusieurs grandeurs d'environnement externe dans le but d'assurer la correction de l'intervalle de temps mesuré en fonction de ladite grandeur d'environnement.

**6.** Dispositif selon la revendication 5, dans lequel ledit boîtier est aussi équipé d'un support pouvant être déplacé verticalement avec au moins deux détecteurs sous la forme d'électrodes, qui détectent le niveau de la surface de mousse selon le principe de conduction électrique, de même que des moyens de déplacement et de positionnement dudit support et desdites électrodes, **caractérisé en ce qu'**un capteur de température est disposé à proximité de l'extrémité libre d'au moins une électrode afin d'enregistrer la température de la couche de mousse.

**7.** Dispositif selon la revendication 5, **caractérisé en ce que** ledit détecteur est sous la forme d'un détecteur optique, qui est disposé à côté de la couche de mousse.

**8.** Dispositif selon la revendication 5, **caractérisé en ce que** ledit détecteur est sous la forme d'un détecteur optique ou d'un détecteur à ultrason, qui est disposé au-dessus de la couche de mousse.

**9.** Dispositif selon l'une quelconque des revendications 5 à 8, **caractérisé en ce que** ledit capteur est un capteur de température, un capteur de pression d'air, un capteur d'humidité d'air, ou un capteur de vitesse de circulation d'air.

Fig. 1

Fig. 2a

Fig. 2b